# EUROPEAN PATENT APPLICATION

(11) **EP 4 715 063 A1**
(43) Date of publication of application: **25.03.2026**
(21) Application number: 24806966.8
(22) Date of filing: 19.04.2024
(51) Int. Cl.: C12Q 1/68, C12Q 1/686, C12Q 1/6869, G01N 33/53

(54) **METHOD FOR ACQUIRING COMPOUND HAVING MOLECULAR RECOGNITION ABILITY, METHOD FOR IDENTIFYING SAMPLE, AND TRAINED MODEL FOR PREDICTING COMPOUND HAVING MOLECULAR RECOGNITION ABILITY**

(30) Priority: 16.05.2023 JP 2023080605
(71) Applicant: Konica Minolta, Inc., Tokyo 100-7015 (JP)
(72) Inventor: TATSUMI, Atsuro, Tokyo 100-7015 (JP); ASAI, Yuichiro, Tokyo 100-7015 (JP); YOSHIMURA, Atsushi, Tokyo 100-7015 (JP); OKAMOTO, Ken, Tokyo 100-7015 (JP)
(74) Representative: MFG Patentanwälte Meyer-Wildhagen Meggle-Freund Gerhard PartG mbB
(86) International application number: PCT/JP2024/015564
(87) International publication number: WO 2024/237012

(57) **Abstract**

Provided are: a method for acquiring a compound having an efficient molecular recognition ability; a method for identifying a sample using the compound thus acquired; and a trained model that enables the acquisition of a compound having an efficient molecular recognition ability. This method for acquiring a compound having a molecular recognition ability includes: a step A for bringing a target molecule into contact with a substrate on which candidate compounds are arranged in an array, and detecting an interaction signal between the candidate compounds and the target molecule; and a step B for predicting, on the basis of the interaction signal, a compound having a molecular recognition ability by machine learning.

## Description

### Technical Field

The present invention relates to a method for acquiring a compound having a molecular recognition ability, a method for identifying a sample, and a trained model that predicts a compound having a molecular recognition ability.

### Background Art

By using a compound having a function of recognizing a target molecule (molecular recognition ability), it is possible to inhibit the function of the target molecule or specifically detect the target molecule. Examples of the compound having a molecular recognition ability targeting a biomolecule include an antibody, a nucleic acid aptamer, and a peptide aptamer. Nucleic acid aptamers and peptide aptamers have attracted attention as next-generation target molecule recognition materials from the viewpoints of ease of production, storage stability, and the like. These target molecule recognition materials can recognize different target molecules depending on the sequence of bases or amino acids. Therefore, a highly diverse library can be easily prepared by the difference in the sequence of bases or amino acids. Since the diversity of sequences is enormous, such as about 10¹² to 10¹⁸, it is difficult to evaluate in detail the molecular recognition ability of all compounds having different sequences. Therefore, it is necessary to select a candidate compound bound to a target molecule from a huge variety of compounds having different sequences by some method for detailed evaluation. For example, when it is desired to evaluate the molecular recognition ability of a nucleic acid aptamer, a candidate nucleic acid aptamer sequence can be selected from a nucleic acid library by the systematic evolution of ligands by exponential enrichment (SELEX) method. However, due to the bias caused by the polymerase chain reaction (PCR) performed in the SELEX method and the long time required for the SELEX method, a selection method that does not use the SELEX method has recently been developed, as disclosed in Patent Document 1 and Related Art Document 1. There is also another technique in which a modified nucleic acid is introduced into a nucleic acid library to acquire an aptamer having a high binding capacity by the SELEX method (see Patent Document 2). However, in these methods, it is substantially difficult to select only candidate compounds that bind. Therefore, in these methods it is necessary to test whether candidate compounds exhibit binding to the target molecule by a binding test. As the test for examining the binding capacity, evaluation by enzyme-linked immunosorbent assay (ELISA) or surface plasmon resonance is mainly used. These methods require synthesizing each sequence separately. In addition, these methods are limited in the number of sequences that can be evaluated at one time, ranging from 100 to 1500. On the other hand, a next-generation sequencer is used to acquire candidate sequences, and a huge number of candidate sequences ranging from tens to hundreds of thousands can be acquired. Therefore, in the conventional method, only a portion of the sequences can be subjected to the binding test, and there is a possibility that sequences with high binding capacity are missed.

### Citation List

### Patent Literature

Patent Document 1: Japanese Unexamined Patent Application Publication No. 2018-029601
Patent Document 2: Japanese Translation of PCT International Application Publication No. 2019-527043

### Non-patent Literature

Non-patent Document 1: Saito et al., "Single -Round DNA Aptamer Selection by Combined Use of Capillary Electrophoresis and Next Generation Sequencing: An Aptaomics Approach for Identifying Unique Functional Protein-Binding DNAAptamers", Chemistry - A European Journal, volume 27, Issue 39, 2021, 10058-10067

### Summary of Invention

### Technical Problem

The present invention has been made in view of the above-mentioned situations. The problem to be solved by the present invention is to provide a method for efficiently acquiring a compound having a high molecular recognition ability, and a method for identifying a sample by using the compound acquired by the method. Another problem to be solved by the present invention is to provide a trained model capable of efficiently acquiring a compound having a high molecular recognition ability.

### Solution to Problem

In order to solve the above problems, the present inventors have studied the cause of the above problems and the like. As a result, the present inventors have found that the above problems can be solved by a step A of bringing a substrate on which candidate compounds are aligned in an array into contact with a target molecule to detect interaction signals between the candidate compounds and the target molecule, and a step B of predicting a compound having a molecular recognition ability by machine learning based on the interaction signals. This finding led to the present invention.

That is, the above-described problems are solved by the following means.
1. A method for acquiring a compound having a molecular recognition ability, comprising: a step A of bringing a substrate on which a candidate compound is aligned in an array into contact with a target molecule to detect an interaction signal between the candidate compound and the target molecule; and a step B of predicting a compound having a molecular recognition ability by machine learning based on the interaction signal.
2. The method for acquiring the compound having the molecular recognition ability according to item 1, wherein the step A and the step B are repeated using the compound having the molecular recognition ability predicted in the step B as the candidate compound in the step A.
3. The method for acquiring the compound having the molecular recognition ability according to item 1, wherein the compound having the molecular recognition ability is a nucleic acid or a peptide.
4. The method for acquiring the compound having the molecular recognition ability according to item 1, wherein the compound having the molecular recognition ability is a nucleic acid having a base sequence in a range of 10 to 100 bases.
5. The method for acquiring the compound having the molecular recognition ability according to item 1, wherein the compound having the molecular recognition ability is an artificial nucleic acid or a modified nucleic acid.
6. The method for acquiring the compound having the molecular recognition ability according to item 1, wherein the compound having the molecular recognition ability is a compound that exhibits a change in luminescence due to an interaction with the target molecule.
7. The method for acquiring the compound having the molecular recognition ability according to item 1, wherein the compound having the molecular recognition ability is a compound that contains: a luminescent moiety; and a spacer that does not affect luminescence of the luminescent moiety.
8. A method for identifying a sample comprising identifying the sample by using the compound acquired by the method for acquiring the compound having the molecular recognition ability according to item 1.
9. The method for identifying the sample according to item 8, wherein the sample is identified by machine learning based on a pattern of a luminescent spot.
10. The method for identifying the sample according to item 8, wherein the sample is a biological sample.
11. A trained model that predicts a compound having a molecular recognition ability, the model being generated by machine learning using a characteristic feature of a candidate compound as an explanatory variable and an interaction signal between the candidate compound and a target molecule as an objective variable, the interaction signal being obtained by using a substrate on which the candidate compound is aligned in an array.

### Advantageous Effects of Invention

According to the above-described means of the present invention, it is possible to provide a method for efficiently acquiring a compound having a high molecular recognition ability, and a method for identifying a sample by using the compound acquired by the method. Further, according to the above-mentioned means of the present invention, it is possible to provide a trained model capable of efficiently acquiring a compound having a high molecular recognition ability.

### Brief Description of Drawings

[FIG. 1] This is a flowchart illustrating an example of acquiring a compound having a molecular recognition ability from a nucleic acid aptamer.
[FIG. 2] This shows clustering results in an example of screening of IgE aptamer.
[FIG. 3] This shows a histogram of binding signals in the example of the screening of IgE aptamer.
[FIG. 4A] This is a fluorescence microscopic image in an example of sample identification (reaction pattern when fetal bovine serum is reacted).
[FIG. 4B] This is a fluorescent microscopic image in an example of sample identification (reaction pattern when mouse serum is reacted).
[FIG. 5] This shows fluorescence microscope images in an example of designing a fluorescent dye-introduced aptamer.

### Description of Embodiments

A method for acquiring a compound having a molecular recognition ability according to the present invention is characterized in that the method includes a step A of bringing a substrate on which candidate compounds are aligned in an array into contact with a target molecule to detect interaction signals between the candidate compounds and the target molecule, and a step B of predicting a compound having a molecular recognition ability by machine learning based on the interaction signals. This feature is a technical feature common to or corresponding to the following embodiments.

As an embodiment of the method for acquiring a compound having a molecular recognition ability according to the present invention, it is preferable that the step A and the step B are repeated using the compound having a molecular recognition ability predicted in the step B as the candidate compound in the step A. This makes it easy to acquire a compound having a higher molecular recognition ability.

As an embodiment of the method for acquiring a compound having a molecular recognition ability according to the present invention, it is preferable that the compound having a molecular recognition ability is a nucleic acid or a peptide. This is preferable from the viewpoints of ease of production of the compound, storage stability and the like. A nucleic acid and a peptide are preferable also in that they can be synthesized on a substrate by inkjet technology.

As an embodiment of the method for acquiring a compound having a molecular recognition ability according to the present invention, it is preferable that the compound having a molecular recognition ability is a nucleic acid having a base sequence in a range of 10 to 100 bases. This is preferable from the viewpoint of molecular recognition ability.

As an embodiment of the method for acquiring a compound having a molecular recognition ability according to the present invention, it is preferable that the compound having a molecular recognition ability is an artificial nucleic acid or a modified nucleic acid. This is preferable from the viewpoint of molecular recognition ability.

As an embodiment of the method for acquiring a compound having a molecular recognition ability according to the present invention, it is preferable that the compound having a molecular recognition ability is a compound that exhibits a change in luminescence due to an interaction with the target molecule. This facilitates the simultaneous detection of a large number of target molecules.

As an embodiment of the method for acquiring a compound having a molecular recognition ability according to the present invention, it is preferable that the compound having a molecular recognition ability is a compound that contains a luminescent moiety and a spacer that does not affect luminescence of the luminescent moiety. This is preferable from the viewpoint of ease of detection of interaction signals.

A method for identifying a sample according to the present invention is characterized in that the sample is identified by using the compound acquired by the method for acquiring a compound having a molecular recognition ability according to the present invention.

As an embodiment of the method for identifying a sample according to the present invention, it is preferable that the sample is identified by machine learning based on a pattern of a luminescent spot. This is preferable from the viewpoint of efficiency of the sample identification.

As an embodiment of the method for identifying a sample according to the present invention, it is preferable that the sample is a biological sample. It is generally difficult to identify a biological sample because the biological sample contains a large number of molecules. However, more accurate identification becomes possible by using the identification method according to the present invention.

A trained model that predicts a compound having a molecular recognition ability according to the present invention is characterized in that the trained model has been subjected to machine learning using characteristic features of candidate compounds as explanatory variables and interaction signals between the candidate compounds and a target molecule as objective variables, the interaction signals being obtained by using a substrate on which the candidate compounds are aligned in an array.

Hereinafter, the present invention will be described in detail. In the present description, numerical values described before and after "to" are included in a range as a lower limit value and an upper limit value.

### [Method for Acquiring Compound Having Molecular Recognition Ability]

The method for acquiring a compound having a molecular recognition ability according to the present invention is characterized by including the following step A and step B.

Step A: A substrate on which candidate compounds are aligned in an array is brought into contact with a target molecule, and interaction signals between the candidate compounds and the target molecule are detected.

Step B: Based on the interaction signals, a compound having a molecular recognition ability is predicted by machine learning.

### [Target Molecule]

The type of the target molecule is not particularly limited and may be, for example, a target molecule used in the medical field or a target molecule used in the industrial field.

Examples of the target molecule include the following.
- A molecule that functions as a biomarker enabling disease diagnosis or detection of preclinical conditions through its detection or quantification
- A biomolecule capable of treating a disease or alleviating a symptom of a disease by inhibiting or enhancing its function
- A molecule to be purified or removed in the production of an article

The target molecule according to the present invention is, for example, a molecule contained in a biological sample. Examples of the biological sample include whole blood, leukocytes, peripheral blood mononuclear cells, plasma, serum, sputum, breath, urine, semen, saliva, meningeal fluid, amniotic fluid, glandular fluid, lymphatic fluid, nipple aspirate, bronchial aspirate, synovial fluid, joint aspirate, cells, cultures of cells, cell extracts, stool, tissue extracts, and cerebrospinal fluid. The target molecule may not be a biomolecule such as a protein and may be a low molecular weight compound, a polymer, or the like.

### [Compound Having Molecular Recognition Ability]

The term "compound having a molecular recognition ability" refers to a compound having a function of recognizing the target molecule. The compound can inhibit the function of the target molecule or specifically detect the target molecule.

The term "candidate compound" refers to a compound that is a candidate for the compound having a high molecular recognition ability to be finally acquired.

The compound having a molecular recognition ability according to the present invention may be, for example, an antibody, a nucleic acid, or a peptide. The compound having a molecular recognition ability is preferably a nucleic acid or a peptide from the viewpoints of ease of production, storage stability, and the like. A nucleic acid and a peptide are preferable also in that they can be synthesized on a substrate by inkjet technology.

Examples of the nucleic acid include modified nucleic acids and artificial nucleic acids. Examples of the modified or artificial nucleic acids include the following.
- Locked nucleic acids (LNA)
- Bridged nucleic acids (BNA)
- Phosphorothianates
- 2'-OMe, amido-bridged nucleic acids (AmNA)

It is preferable that the compound having a molecular recognition ability is a nucleic acid having a base sequence in the range of 10 to 100 bases from the viewpoint of molecular recognition ability.

The compound having a molecular recognition ability may partially contain a low molecular weight compound or a low molecular weight functional group, or an artificially created compound. Examples of the low molecular weight compound or the low molecular weight functional group include biotin and an amino group. Examples of the artificially created compound include a modified nucleic acid, an artificial nucleic acid, an artificial amino acid, and a luminescent dye. Among these, the compound having a molecular recognition ability is preferably an artificial nucleic acid or a modified nucleic acid from the viewpoint of molecular recognition ability.

The compound having a molecular recognition ability is preferably a compound that exhibits a change in luminescence due to an interaction with the target molecule. This facilitates the simultaneous detection of a large number of target molecules. The term "luminescence" in the present invention includes fluorescence and phosphorescence. The compound that exhibits a change in luminescence due to an interaction with the target molecule contains a luminescent moiety derived from, for example, a luminescent dye.

The luminescent dye may be pyrene, which is a luminescent dye having the luminescent properties of a monomer or an excimer, or a combination of a luminescent dye and a quencher used in a molecular beacon. Examples of a fluorescent dye include 6-FAM, JOE, TET, HEX, Cy3, ROX, Texas Red, Cy5, and Cy5.5. Examples of the quencher include BHQ-1, BHQ-2, BHQ-3, and DABCYL. These fluorescent dye and quencher may be substituted, for example, at a base site of a nucleic acid.

The compound having a molecular recognition ability is more preferably a compound that contains a luminescent moiety and a spacer that does not affect luminescence of the luminescent moiety. This makes it easier to detect the interaction signals. Examples of the spacer include an alkyl chain, a sugar backbone portion of a nucleic acid, polyethylene glycol, and a peptide.

In the present invention, when the compound or candidate compound having a molecular recognition ability is a nucleic acid, it is also referred to as a "nucleic acid aptamer". Similarly, when the compound or candidate compound having a molecular recognition ability is a peptide, it is also referred to as a "peptide aptamer".

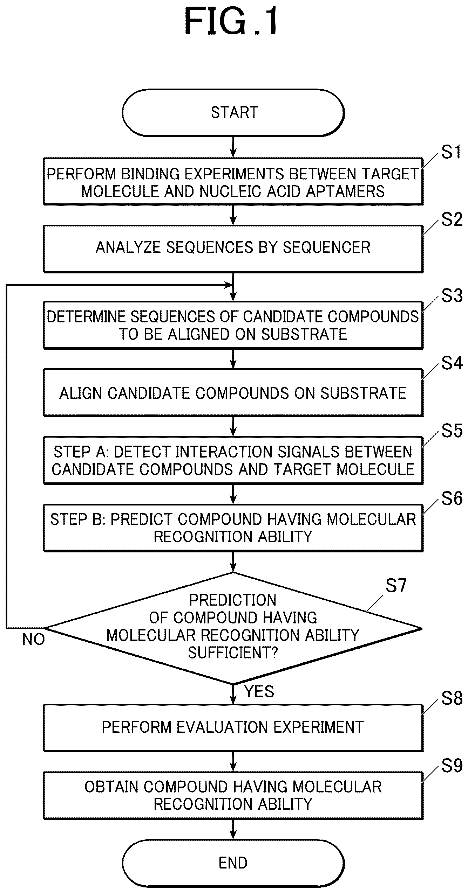

Hereinafter, the method for acquiring a compound having a molecular recognition ability will be described with reference to a flowchart illustrated in FIG. 1. FIG. 1 is a flowchart exemplifying a case where the compound having a molecular recognition ability is acquired from nucleic acid aptamers. It is only required that the present invention includes the step A and the step B. The other steps illustrated in the flowchart of FIG. 1 do not limit the present invention.

In S1, binding experiments between the target molecule and nucleic acid aptamers are performed. This narrows down the number of nucleic acid aptamers confirmed to bind to the target molecule to about several hundred thousand.

In S2, the sequence of each nucleic acid aptamer confirmed to bind to the target molecule in the binding experiment of S1 is analyzed by a sequencer.

One embodiment of S1 and S2 will be described by exemplifying the case where the compound having a molecular recognition ability is acquired from nucleic acid aptamers. For candidate sequences of nucleic acid aptamers, sequences capable of binding to the target molecule can be selected by the SELEX method. As a method for selecting the nucleic acid sequences capable of binding to the target molecule, a general-purpose method for acquiring aptamer candidate sequences, such as the SELEX method, can be employed. For example, a nucleic acid library including a primer region and a random region is mixed with magnetic beads on which the target molecule is immobilized, and nucleic acid sequences capable of binding to the target molecule are isolated. From the complex of the nucleic acids and the magnetic beads of the immobilized target molecule, only the nucleic acids are isolated by a solution containing sodium hydroxide or heat treatment. The isolated nucleic acids are used as a template for PCR amplification. The product is then denatured into single strands by treatment with a sodium hydroxide solution or heat, serving as a library for the next round. By repeating this step, the nucleic acid sequences capable of binding to the target molecule are enriched. The nucleic acid sequences capable of binding to the target molecule are obtained by analyzing the enriched sequences using a next-generation sequencer. The immobilization of the target molecule is not limited to magnetic beads, and a general carrier such as a membrane, a gel, or a resin can be employed. The nucleic acids capable of binding to the target molecule may be selected without immobilizing the target molecule. For example, a nucleic acid library and the target molecule may be mixed and subjected to an ultrafiltration filter to isolate the nucleic acid sequences capable of binding to the target molecule. Alternatively, for example, a sample in which a nucleic acid library and the target molecule are mixed may be subjected to capillary electrophoresis for isolation. Note that the method for selecting these nucleic acids is not limited to the SELEX method. The nucleic acid sequences capable of binding to the target molecule may be selected without the step of enriching the library by PCR amplification. In addition, the sequence analysis is not limited to the next-generation sequencer, and a nanopore sequencer may be used.

In S3, the sequences of candidate compounds to be aligned on a substrate are determined. As the sequences of the candidate compounds to be aligned on the substrate, any sequence may be selected from the sequences identified by a sequencer or the like. In addition, the sequences of the candidate compounds to be aligned on the substrate may be selected using a machine learning method such as unsupervised learning, semi-supervised learning, or supervised learning. Hereinafter, an example of selection using a machine learning method will be described.

### (Example of Selection of Sequences of Candidate Compounds Using Unsupervised Machine Learning Model)

By training a suitable unsupervised machine learning model using information on the identified sequences or the like as an explanatory variable, compounds can be grouped for similar sequences. Examples of the unsupervised machine learning model include k-means clustering, principal component analysis, and variational auto encoder (VAE), which is a deep learning model. When the VAE is trained on the identified sequences, the VAE becomes capable of encoding (converting) the sequence information into numeric values of a specified number of dimensions (latent space). See Nature Computational Science volume 2, pages 378-386, 2022. In this step, the numerical values in the latent space are expected to be similar between similar sequences, in particular, between sequences having characteristic partial sequences (characteristic sequences). Therefore, by selecting sequences having similar numerical values in the latent space, it is also possible to select a group of sequences having a particular partial sequence. Conversely, by selecting sequences in such a way that the numerical values in the latent space are as varied as possible, it is also possible to increase the variation in a group of sequences to be selected. As a means for varying the numerical values in the latent space for the sequences to be selected, a D-optimal programming method or the like is exemplified. The trained unsupervised machine learning model can predict the clustering patterns and latent space for not only the trained sequences but also any untrained sequence. Thus, a sequence that has not been trained by the machine learning model can also be targeted for selection of the candidate compounds to be aligned on the substrate.

### (Example of Selection of Sequences of Candidate Compounds Using Supervised Machine Learning Model)

The sequences of the candidate compounds may be selected with reference to a predicted value of any supervised machine learning model. Examples of the supervised machine learning model include a multiple regression model, a PLS regression model, and a deep learning model. For example, a supervised machine learning model is trained using interaction signals of the target molecule with respective sequences as objective variables and sequence information as an explanatory variable. Then, an interaction signal of the target molecule can be predicted from the sequence information. The machine learning model can predict an interaction signal of the target molecule not only with the trained sequences but also with any untrained sequence. This machine learning model is used to predict an interaction signal of the target molecule with any given sequence, and a sequence having a high prediction value is selected. Thus, a sequence that is highly likely to have a high binding capacity can be efficiently derived.

For the selection of the sequences of the candidate compounds to be aligned on the substrate, any sequence can be considered a candidate, not limited to the sequences identified by the sequencer. The number of sequences to be candidates for the selection may be increased by any method such as generating a sequence by a generative model in which a mutation is added to a sequence using a genetic algorithm or the like. Examples of such generative models include VAE, generative adversarial networks (GAN), FLOW, and a diffusion model. Previously reported sequences may be considered candidates for the selection.

The explanatory variable in the machine learning is not limited, and examples include the sequence information, tertiary structure, and physical property value of any compound. The objective variable is also not limited, and examples include an interaction signal value of the target molecule and a dissociation constant. Both the explanatory variable and the objective variable may include information on previously reported sequences.

In S4, the candidate compounds are aligned in an array on the substrate. In this step, the candidate compounds may be synthesized on the substrate. For the synthesis of the candidate compounds on the substrate, a printing technique such as inkjet or a photolithography technique can be used.

Step S5 is step A. In step A, the substrate on which the candidate compounds are aligned in an array is brought into contact with the target molecule, and interaction signals between the candidate compounds and the target molecule are detected. An interaction signal is a signal generated by an interaction, such as binding, between one of the candidate compounds and the target molecule. The interaction includes a chemical bond, an intermolecular interaction, and the like. The interaction signal generated by a chemical bond is also referred to as a binding signal. The interaction signal is, for example, luminescence generated by the binding of the candidate compound to the target molecule. The luminescence includes fluorescence and phosphorescence.

In one example of step A, first, a solution containing the target molecule is added dropwise to the substrate on which the candidate compounds are aligned in an array. The candidate compound in each spot is reacted with the target molecule, and an interaction signal between the candidate compound and the target molecule is detected.

Examples of a method for detecting the interaction signal include an indirect fluorescence method using an antibody labeled with a fluorescent dye, and a method for designing and detecting a candidate compound that changes fluorescence upon binding to the target molecule.

Step S6 is step B. In step B, a compound having a molecular recognition ability is predicted by machine learning based on the interaction signals detected in step A. Specifically, a trained model generated by machine learning predicts a compound having a molecular recognition ability based on input interaction signals, and outputs the predicted compound.

The trained model that predicts a compound having a molecular recognition ability can be generated by, for example, training a learning model by machine learning using characteristic features of the candidate compounds as explanatory variables and interaction signals between the candidate compounds and the target molecule as objective variables, the interaction signals being obtained by using the substrate on which the candidate compounds are aligned in an array. The characteristic features of the candidate compound include, for example, the base sequence, tertiary structure, physical property value, and the like of the nucleic acid aptamer as the candidate compound.

In S7, it is determined whether the prediction of the compound having a molecular recognition ability in step B is sufficient. If the prediction of the compound having a molecular recognition ability is sufficient, the process proceeds to an evaluation experiment in S8.

If the prediction of the compound having a molecular recognition ability is not sufficient, the process returns to S3 in order to perform step A and step B again. In this step, the steps A and B may be repeated using the compound having a molecular recognition ability predicted in step B as the candidate compound in step A.

In S8, an evaluation experiment is performed on the compound having a molecular recognition ability predicted in step B. The evaluation experiment is, for example, a binding experiment with the target molecule.

In S9, the compound having a molecular recognition ability is acquired from the results of the evaluation experiment in S8.

### [Method for Identifying Sample]

The compound acquired by the method for acquiring a compound having a molecular recognition ability according to the present invention can be used for identifying a sample.

The sample to be identified is, for example, a biological sample. Examples of the biological sample include whole blood, leukocytes, peripheral blood mononuclear cells, plasma, serum, sputum, breath, urine, semen, saliva, meningeal fluid, amniotic fluid, glandular fluid, lymphatic fluid, nipple aspirate, bronchial aspirate, synovial fluid, joint aspirate, cells, cultures of cells, cell extracts, stool, tissue extracts, and cerebrospinal fluid.

A biological sample is a mixture of numerous molecules. If compounds having a molecular recognition ability for numerous molecules can be acquired, for a complex sample in which numerous molecules are present, it will be possible to identify the sample more accurately. Among the numerous molecules, a molecule that can be a target molecule may be a molecule that has already been identified or a molecule that has not been identified.

One embodiment of the method for identifying a sample includes the following steps.
(1) Step of selecting a compound having a molecular recognition ability capable of identifying the sample
(2) Step of synthesizing the compound on a substrate by inkjet technology
(3) Step of bringing the sample into contact with the substrate
(4) Step of obtaining a fluorescent image
(5) Step of analyzing the fluorescent image

The whole molecules in the sample may be labeled with biotin, a luminescent dye, or the like. In this step, the sample may be identified by machine learning based on a pattern of observed luminescent spots.

### [Examples]

Hereinafter, the present invention will be specifically described with reference to Examples, but the present invention is not limited thereto. Note that in the following Examples, operations were performed at room temperature (25°C) unless otherwise specified. Further, unless otherwise specified, "%" and "parts" mean "% by mass" and "parts by mass", respectively.

### [Screening of IgE Aptamer]

IgE-immobilized magnetic beads and a DNA library were mixed and stirred for 1 hour, and then the supernatant was removed using a magnetic stand. Thereafter, the magnetic beads were washed with a phosphate buffer solution containing Tween 20. Thereafter, an aqueous sodium hydroxide solution of 0.15 N was added dropwise to dissociate the DNA from the magnetic beads. The mixture was placed on a magnetic stand to collect the supernatant. The collected solution was neutralized by dropwise addition of an aqueous HCl solution of 1 N. This sample was collected as the DNA considered to bind to the target molecule. The collected DNA was subjected to PCR amplification using a biotin-labeled primer and then to single-strand preparation, thereby preparing a library for the next round. This step was repeated for three rounds, and then the sequences were analyzed with a next-generation sequencer.

### (Sequence Identification by Next-generation Sequencer)

The fractions after screening were processed with the NEBNext (registered trademark) Multiplex Oligos for Illumina kit. Thereafter, this was subjected to MiSeq to identify the sequences contained in the fractions.

### (Sequence Selection by Unsupervised Learning)

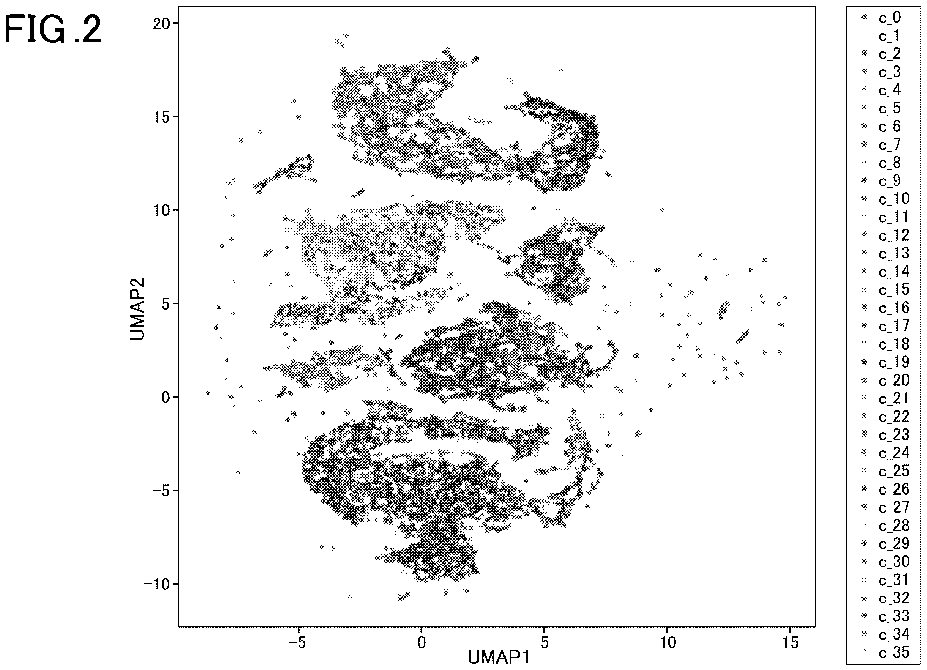

A VAE model, which is a type of unsupervised machine learning model, was trained using the sequences identified by the next-generation sequencer as explanatory variables. This converted the data into 10-dimensional numbers. In this step, similar sequences are converted into similar numbers. These numbers were subjected to GMM clustering, which is a type of unsupervised machine learning model, to classify similar sequences. FIG. 2 shows the results of compressing 10-dimensional numbers into two dimensions by the UMAP method and coloring the compressed numbers according to the clustering results. Based on the results, the sequences of the candidate compounds to be immobilized on a substrate were selected.

A microarray in which the candidate compounds were immobilized in an array on a substrate was obtained from Agilent Technologies. 2% bovine serum albumin (BSA) was added dropwise to the microarray and allowed to stand for 30 minutes for blocking. Next, the microarray was washed with a phosphate buffer solution containing Tween 20. Thereafter, an IgE solution of 100 nM was applied to the microarray and allowed to stand at 25°C for 1 hour. Next, the microarray was washed with a phosphate buffer solution containing Tween 20. Thereafter, a fluorescein-labeled anti-IgE antibody was applied to the microarray and allowed to stand at 25°C for 1 hour. Then, unreacted fluorescein-labeled anti-IgE antibody was washed with a phosphate buffer solution containing Tween 20 and sealed with a phosphate buffer solution and a cover glass. Thereafter, fluorescence of the fluorescein was photographed by a fluorescence microscope. Next, the photographed image was subjected to image analysis by machine learning to quantify the fluorescence intensity of each spot as a binding signal. The binding signal value quantified in this step is defined as the value of Round 1.

### (Machine Learning (Regression Model))

A deep regression model was trained with each sequence immobilized on the microarray as an explanatory variable and its binding signal value as an objective variable. The deep regression model was given suitable sequences as explanatory variables to predict their binding signal values.

Sequences with high predicted binding signals were selected. Compounds of the selected sequences were synthesized on a microarray. A binding signal value of the target molecule with each synthesized compound was quantified by a binding experiment. The binding signal value quantified in this step is defined as the value of Round 2.

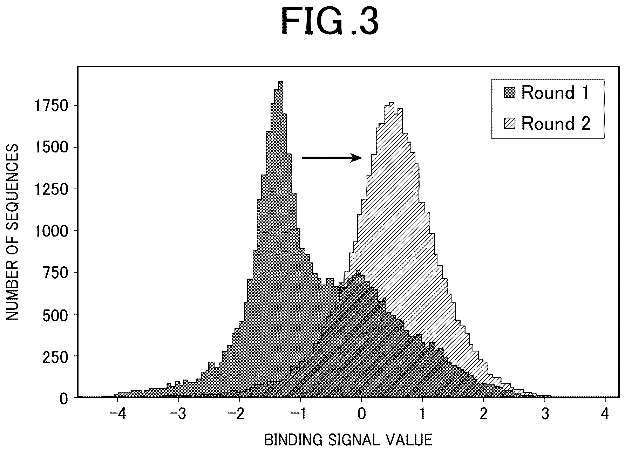

As illustrated in histogram of FIG. 3 and Table I, both the mean value and the maximum value of the binding signals were improved from Round 1 to Round 2. Accordingly, it was confirmed that a compound having a higher molecular recognition ability can be acquired by the present invention.

### [Table 1]

**TABLE I**

| | Round 1 | Round 2 |
|---|---|---|
| MEAN VA_LE | -0.75 | 0.5 |
| MAX MUM VA_UE | 2.82 | 3.84 |

### [Identification of Sample]

Albumin and globulin contained in fetal bovine serum and mouse serum were removed. For the removal of albumin and globulin, an Aurum Affi-Gel (Bio Rad) bead purification kit was used. Thereafter, each sample was labeled with biotin, mixed with a DNA library, and subjected to the SELEX method. Based on sequences analyzed by a next-generation sequencer, candidate compounds having a molecular recognition ability were screened respectively from the fetal bovine serum and the mouse serum. The candidate compounds were synthesized so as to be aligned in an array on a substrate.

Biotin-labeled fetal bovine serum or mouse serum was applied to this substrate. Unbound fetal bovine serum or mouse serum was washed off. Thereafter, fluorescein-labeled streptavidin was applied to the substrate. Unbound fluorescein-labeled streptavidin was washed off.

Thereafter, fluorescent spots emitting light by fluorescein were photographed with a fluorescence microscope. The photographed image was subjected to image analysis to detect binding signals.

Based on the binding signals, sequences having a high binding capacity were predicted by machine learning. Compounds having the predicted sequences were synthesized so as to be aligned in an array on a substrate.

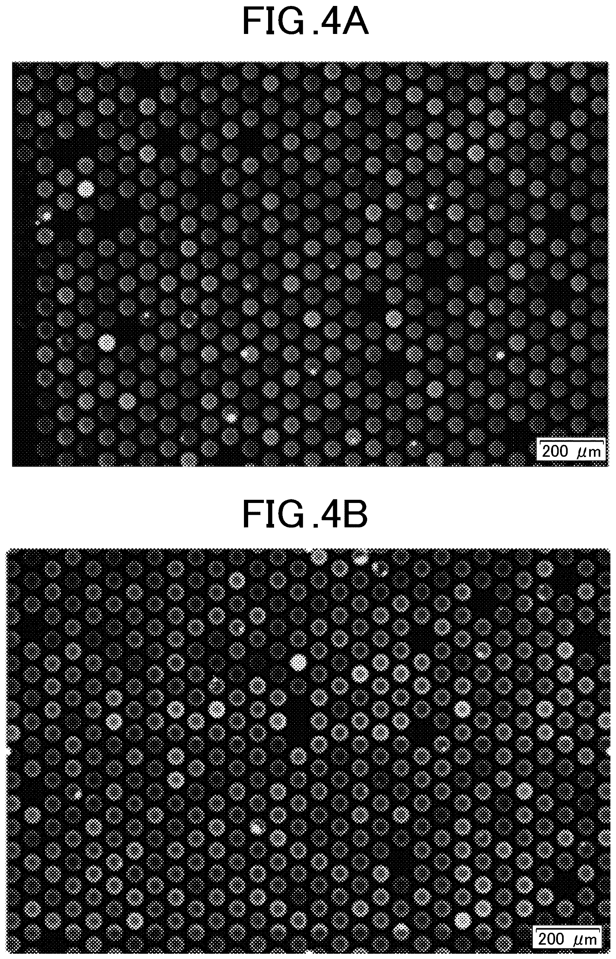

Biotin-labeled fetal bovine serum or biotin-labeled mouse serum was dropped onto this substrate and allowed to stand at 25°C for 1 hour. After washing the substrate, a fluorescein-labeled streptavidin solution was added dropwise and allowed to stand at 25°C for 1 hour. After washing the substrate, the array was photographed using a fluorescence microscope. As a result of analyzing the photographed images, different reaction patterns were observed when the fetal bovine serum was reacted and when the mouse serum was reacted. The reaction pattern when the fetal bovine serum was reacted is illustrated in FIG. 4A, and the reaction pattern when the mouse serum was reacted is illustrated in FIG. 4B.

This result confirms that it is possible to identify differences between bovine and mouse sera by acquiring a variety of aptamers for the sample to be identified.

### [Design of Fluorescent Dye-introduced Aptamer]

Oligonucleotides with pyrene-substituted bases were inserted at both ends of a thrombin-binding aptamer. A spacer composed of ten base-deficient sugar backbones was inserted at the 3'-terminus of this aptamer, and an amino group was modified at the 3'-terminus. Thus, an aptamer into which a fluorescent dye was introduced was designed. This aptamer was immobilized on a glass substrate by an amine coupling method. A thrombin solution or a buffer solution was dropped on the substrate, and a cover glass was placed thereon and allowed to stand at 25°C for 30 minutes.

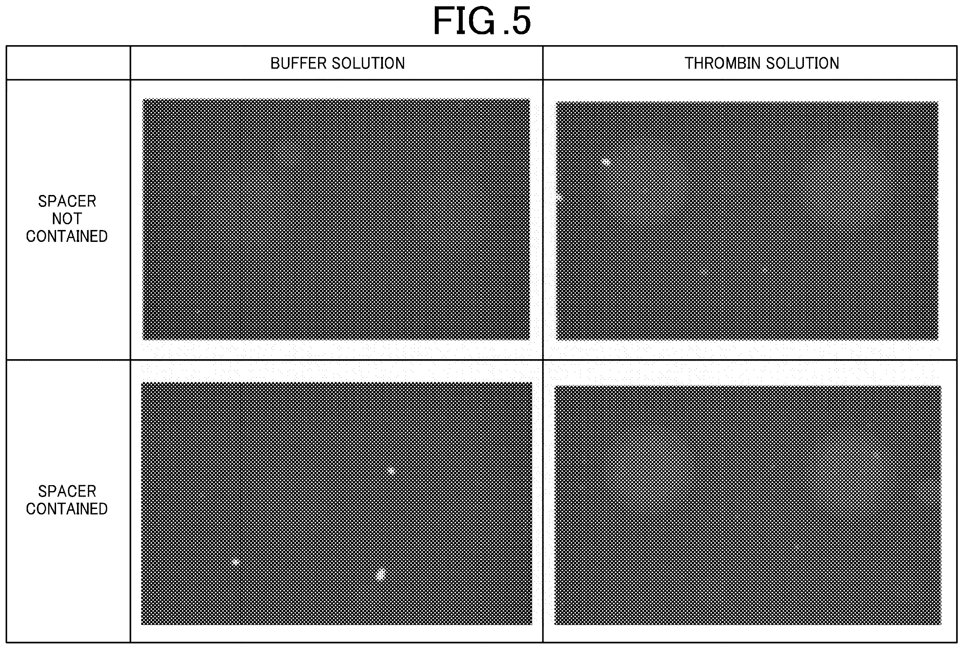

The immobilized portion was observed with a fluorescence microscope. FIG. 5 shows fluorescence microscope images of the results of observation.

Almost no fluorescent spot was observed in the immobilized portion to which the buffer solution was dropped. Fluorescent spots considered to be excimer luminescence were observed in the immobilized portion to which the thrombin solution was dropped. This result confirms that the structure of the aptamer was changed with the binding to thrombin.

When the spacer was not contained, fluorescent spots were also observed in the immobilized portion to which the buffer solution was dropped. This result confirms that the insertion of the spacer can reduce the background, enabling high-sensitivity detection of thrombin.

### Industrial Applicability

The present invention can be used for a method for acquiring a compound having a molecular recognition ability, a method for identifying a sample, and a trained model that predicts a compound having a molecular recognition ability.

## Claims

1. A method for acquiring a compound having a molecular recognition ability, comprising:
a step A of bringing a substrate on which a candidate compound is aligned in an array into contact with a target molecule to detect an interaction signal between the candidate compound and the target molecule; and
a step B of predicting a compound having a molecular recognition ability by machine learning based on the interaction signal.

2. The method for acquiring the compound having the molecular recognition ability according to claim 1, wherein the step A and the step B are repeated using the compound having the molecular recognition ability predicted in the step B as the candidate compound in the step A.

3. The method for acquiring the compound having the molecular recognition ability according to claim 1, wherein the compound having the molecular recognition ability is a nucleic acid or a peptide.

4. The method for acquiring the compound having the molecular recognition ability according to claim 1, wherein the compound having the molecular recognition ability is a nucleic acid having a base sequence in a range of 10 to 100 bases.

5. The method for acquiring the compound having the molecular recognition ability according to claim 1, wherein the compound having the molecular recognition ability is an artificial nucleic acid or a modified nucleic acid.

6. The method for acquiring the compound having the molecular recognition ability according to claim 1, wherein the compound having the molecular recognition ability is a compound that exhibits a change in luminescence due to an interaction with the target molecule.

7. The method for acquiring the compound having the molecular recognition ability according to claim 1, wherein the compound having the molecular recognition ability is a compound that contains:
a luminescent moiety; and
a spacer that does not affect luminescence of the luminescent moiety.

8. A method for identifying a sample, comprising identifying the sample by using the compound acquired by the method for acquiring the compound having the molecular recognition ability according to claim 1.

9. The method for identifying the sample according to claim 8, wherein the sample is identified by machine learning based on a pattern of a luminescent spot.

10. The method for identifying the sample according to claim 8, wherein the sample is a biological sample.

11. A trained model that predicts a compound having a molecular recognition ability, the model being generated by machine learning using a characteristic feature of a candidate compound as an explanatory variable and an interaction signal between the candidate compound and a target molecule as an objective variable, the interaction signal being obtained by using a substrate on which the candidate compound is aligned in an array.
